(19) Europäisches Patentamt
European Patent Office
Office européen des brevets

(11) EP 3 177 687 B1

(12) **EUROPEAN PATENT SPECIFICATION**

(45) Date of publication and mention of the grant of the patent:
**30.08.2023 Bulletin 2023/35**

(21) Application number: **15753184.9**

(22) Date of filing: **02.07.2015**

(51) International Patent Classification (IPC):
***C09D 5/14*** (2006.01) ***A61F 6/04*** (2006.01)
***A61L 27/16*** (2006.01) ***A61L 27/34*** (2006.01)
***A61L 29/08*** (2006.01) ***A61L 29/16*** (2006.01)
***A61L 31/04*** (2006.01) ***A61L 31/10*** (2006.01)
***A61L 26/00*** (2006.01) ***C08K 5/00*** (2006.01)
***C08L 9/06*** (2006.01) ***C09D 109/06*** (2006.01)
***C09D 127/06*** (2006.01) ***C08L 27/06*** (2006.01)

(52) Cooperative Patent Classification (CPC):
(C-Sets available)
**A61L 31/10; A61F 6/04; A61L 29/085; A61L 29/16; A61L 31/16; C08K 5/1535; C09D 5/14; C09D 127/06; C09D 153/025;** A61L 2300/404; A61L 2300/412 (Cont.)

(86) International application number:
**PCT/IB2015/054985**

(87) International publication number:
**WO 2016/020774 (11.02.2016 Gazette 2016/06)**

(54) **ELASTOMERIC COMPOSITIONS COMPRISING USNIC ACID AND DEVICES MADE THEREOF OR COATED THEREWITH**

ELASTOMERZUSAMMENSETZUNGEN MIT USNINSÄURE UND DARAUS HERGESTELLTE ODER DAMIT BESCHICHTETE VORRICHTUNGEN

COMPOSITIONS ÉLASTOMÈRES COMPRENANT DE L'ACIDE USNIQUE ET DISPOSITIFS FABRIQUÉS À PARTIR DE CELLES-CI OU ENROBÉS DE CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2014 IT MI20141444**

(43) Date of publication of application:
**14.06.2017 Bulletin 2017/24**

(73) Proprietor: **Apharm S.r.l.**
**28041 Arona (NO) (IT)**

(72) Inventors:
- **GUERRA, Emanuele**
  **I-25062 Concesio (IT)**
- **PIZZONI, Angelo**
  **I-28041 Arona (NO) (IT)**
- **POZZI, Paolo**
  **I-28041 Arona (NO) (IT)**

(74) Representative: **Trupiano, Federica**
**Marietti, Gislon e Trupiano S.r.l.**
**Via Larga, 16**
**20122 Milano (IT)**

(56) References cited:
**EP-A1- 0 141 628**
**WO-A1-90/05551**
**WO-A1-2012/123803**
**FR-A1- 2 910 015**
**US-A- 5 804 628**
**US-A1- 2011 300 202**
**US-A1- 2014 170 238**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **FRANCOLINI I ET AL: "Usnic acid, a Natural Antimicrobial Agent Able to Inhibit Bacterial Biofilm Formation on Polymer Surfaces", ANTIMICROBIAL AGENTS AND CHEMOTHERAPY, AMERICAN SOCIETY FOR MICROBIOLOGY, vol. 48, no. 11, 1 November 2004 (2004-11-01), pages 4360-4365, XP002660483, ISSN: 0066-4804, DOI: 10.1128/AAC.48.11.4360-4365.2004**
- **LABIB M E ET AL: "The long-term release of antibiotics from monolithic nonporous polymer implants for use as tympanostomy tubes", COLLOIDS AND SURFACES. A, PHYSICACHEMICAL AND ENGINEERING ASPECTS, ELSEVIER, AMSTERDAM, NL, vol. 354, no. 1-3, 5 February 2010 (2010-02-05), pages 331-337, XP026824932, ISSN: 0927-7757 [retrieved on 2009-11-10]**
- **MARTHA MERCHAN ET AL: "Thermoplastic modification of medical grade polyvinyl chloride with various antibiotics: effect of antibiotic chemical structure on mechanical, antibacterial properties, and release activity", POLYMER BULLETIN, SPRINGER, BERLIN, DE, vol. 67, no. 6, 20 March 2011 (2011-03-20) , pages 997-1016, XP019942948, ISSN: 1436-2449, DOI: 10.1007/S00289-011-0474-3**
- **L.R. GOODES ET AL: "Estimation of organic biocide leaching rate using a modified cavity jump diffusion model", PROGRESS IN ORGANIC COATINGS, vol. 77, no. 9, 15 May 2014 (2014-05-15), pages 1499-1505, XP055171913, ISSN: 0300-9440, DOI: 10.1016/j.porgcoat.2014.04.016**
- **R. BENGÜ KARABACAK ET AL: "Preparation of novel antimicrobial polymer colloids based on (+)-usnic acid and poly(vinylbenzyl chloride)", REACTIVE & FUNCTIONAL POLYMERS, vol. 83, 12 July 2014 (2014-07-12), pages 7-13, XP055171903, ISSN: 1381-5148, DOI: 10.1016/j.reactfunctpolym.2014.07.002**


(52) Cooperative Patent Classification (CPC): (Cont.)

C-Sets

**A61L 29/085, C08L 53/02;**
**A61L 31/10, C08L 53/02;**
**C08K 5/1535, C08L 27/06;**
**C08K 5/1535, C08L 53/025;**
**C09D 127/06, C08K 5/1535;**
**C09D 153/025, C08K 5/1535**

## Description

### Summary of the Invention

The present invention relates to elastomeric compositions useful for making and/or coating medicated devices. The invention also relates to such devices and to a process for making and using them.

### Technical Field

Usnic acid, or 2,6-diacetyl-7,9-dihydroxy-8,9b-dimethyl-1,3(2H,9bh)-dibenzofurandione, having the formula of the following structure

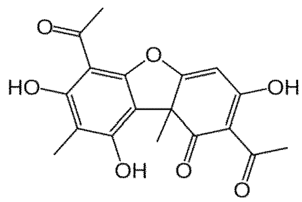

is a known compound, originally extracted from some lichen species, presenting antimicrobial and healing properties.

Devices prepared starting from, or also simply coated with, elastomeric mixtures comprising polymers or copolymers and various additives, are known.

By way of example, patches and condoms, but also devices such as patches, catheters and the likes, made with copolymers such as "styrene-ethylenebutylene-styrene" (SEBS) copolymer or "styrene butadiene compound" (SBC) copolymer, can be mentioned. Such devices are generally prepared by using various methods, for example by dipping punches in solutions comprising said (co)polymers, according to "dipping" technique.

Some of these devices are used as protective barrier for the wounds, as in case of patches or for personal protection against contact to people or things, as in case of gloves and condoms, or also as medical devices such as for example catheters, drainage tubes of wounds, infusion tubes of pharmaceutical compositions, etc.

It is understood that such devices necessarily constitute a really effective protection and, where necessary, that such devices are as bacteriologically pure as possible, to limit the risk of contaminations.

Francolini et al. Antimicrobial Agents and Chemotherapy, Nov. 2004, 4360-4365, describe compositions in which usnic acid is reacted with chemically modified polyurethane polymers, namely polyurethanes in which tertiary amine groups have been introduced, in order to bind the acid groups of the usnic acid. WO2012/123803 discloses PVC catheters which are coated by a composition, made by a sol-gel technique, comprising usnic acid, i. e an article which is made by two distinct parts, i.e. a PVC containing portion and a coating layer comprising usnic acid.

### Objects of the Invention

It is an object of the invention to provide elastomeric compositions suitable for making, or coating, medicated and medical devices intended for the protection against pathogenic microorganisms.

It is another object of the invention to provide elastomeric compositions comprising usnic acid in the extruded-solid or liquid form.

It is another object of the invention to provide medicated devices based on elastomeric compositions comprising usnic acid and a process for preparing them.

### Brief Description of the Figures

Figure 1 schematically shows an example of process according to the invention.
Figure 2 schematically shows another example of process according to the invention.
Figures 3 to 7 show the results of the in vitro assays carried out on a device representative of the invention.

### Abbreviations

The following abbreviations can be used in the present description:

SBS: Polystyrene-b-poly(butadiene)-b-polystyrene
SEP : Polystyrene-b-poly(ethylene/propylene)
SEPS : Polystyrene-b-poly(ethylene/propylene)-b-polystyrene
SEBS : Polystyrene-b-poly(ethylene/butylene)-b-polystyrene
SEEPS: Polystyrene-b-poly(ethylene-ethylene/propylene)-b-polystyrene

In the present description, the expression "b-polystyrene copolymer" denotes any copolymer of the whole series of polymers shown above as well as their possible combinations, in any mutual percentage.

SBC: copolymer composed of styrene-butadiene
THF: tetrahydrofuran
PVC: polyvinylchloride
CFU: Colony Forming Units

## Description of the Invention

The invention refers to novel elastomeric liquid or extruded-solid compositions comprising usnic acid useful for making, or coating, medicated and medical devices, in particular compositions comprising polymers associated with usnic acid. The invention also refers to medicated devices prepared starting from the above referred compositions and a process for making them.

According to the present description, the term "polymer" comprises PVC and copolymers.

According to one of its aspects, subject-matter of the invention are liquid or extruded-solid compositions comprising usnic acid and a polymer selected from PVC and copolymers selected from SBS, SEBS, SEP, SEPS, SEEPS, SBC, mixtures of said copolymers, in a solid form, for example in the form of granules (also called pellets or compounds) or films, or else in solution in an organic solvent.

Unless otherwise stated, with the term "copolymer" herein is meant a copolymer selected from a b-polystyrene copolymer (as defined above), SBC ("styrene butadiene compound" copolymer) and mixtures thereof.

The b-polystyrene copolymer, as defined above, is a copolymer known in the art and represents a preferred copolymer according to the invention. A particularly preferred b-polystyrene copolymer is SEEPS.

The copolymer of the invention advantageously has average molecular weight between 70,000 and 350,000 Dalton, measured according to osmometry technique. However, different molecular weights can be used depending on the type of device to be made and one skilled in the art is able to evaluate which the type of copolymer is more suitable for the different devices.

By way of example, when a low molecular weight copolymer is intended to be used, for example the product currently marketed with the name of SEPTON 4033 is used, whereas when a medium molecular weight copolymer is intended to be used, for example the product currently marketed with the name of SEPTON 4055 is used, both by the Kuraray Company.

Preferably, but not necessarily, the b-polystyrene copolymer has a styrene content between 20 and 70%, for example between 25 and 40%, for example about or equal to 30%.

In alternative or in addition to b-polystyrene copolymer, it is possible to use a SBC copolymer.

The addition of such a copolymer modifies the hardness of the final semi-finished product, and can arrive up to 70 ShD when 100% SBC is used.

As mentioned, another polymer usable for the invention is PVC, for example plasticized PVC.

The Applicant has also tested other polymers for the preparation of the compositions of the invention and of the devices produced, such as for example polyurethanes, but the desired results has not been obtained. Data relative to these experimentations are set forth in the Experimental Section of the present description, together with tests carried out on the compositions of the invention. Usnic acid is known in the art, as well as its antimicrobial activity, against bacteria, yeasts, fungi and other pathogenic microorganisms, as well as its antiviral and anti-inflammatory activity is known.

Unless otherwise stated, all the percentages shown in the present description and in the claims are expressed by weight.

For the preparation of the compositions in a extruded-solid form, for example in granules or films, the selected polymer is mixed with usnic acid and the mixture is extruded to form said solid mixture, at a temperature lower than the melting point of the usnic acid, advantageously at a temperature equal to or lower than 200°C.

The extrusion is accomplished in a conventional way, for example in conventional extruders, for example screw extruders, thereby obtaining the composition in the desired solid form.

The extrusion process of the mixture of the usnic acid with a polymer is a further subject-matter of the invention.

For the preparation of the compositions in a liquid form, it is possible to disperse the extruded composition, as

reported above, in the selected organic solvent or, in alternative, simply to dissolve the usnic acid and the polymer in said solvent. In particular, the invention concerns a process for preparing a liquid composition according to the invention, said process comprising mixing granules of the selected copolymer, advantageously of a styrene copolymer, in the solvent, advantageously, but not exclusively, THF and the adding usnic acid and the possible one or more additives. Alternately, the invention also concerns a process for preparing a liquid composition according to the invention, comprising dispersing the extruded composition, as reported above, in the selected organic solvent and adding the possible additives.

The solvent usable for the compositions of the invention is an organic solvent which allows to dissolve the used polymer and disperse the usnic acid, giving rise to a homogeneous and viscous mixture.

Usable solvents according to the invention include THF, toluene, cyclohexanone, chloroform and dichloromethane.

According to a preferred embodiment, the solvent is tetrahydrofuran (THF). According to another embodiment, the solvent is selected from cyclohexanone and toluene.

When PVC is used as polymer, according to the invention, THF and cyclohexanone are the preferred solvents.

The percentage amounts of the components of the composition can vary depending on the desired antimicrobial efficacy and, for the liquid compositions, the desired viscosity. By way of example, the compositions of the invention in a liquid form comprise 1% to 40% polymer, preferably 2 to 35%, even more preferably 5 to 30% polymer, (advantageously b-polystyrene copolymer, preferably SEEPS), said percentages being by weight with respect to the total weight of the liquid composition.

The compositions in a solid form, for example in granules or films, can comprise 90 to 99% polymer, for example 90-98.5% polymer, with respect to the total weight of the composition.

Still by way of example, the compositions of the invention comprise 0.1 to 30%, preferably 0.5 to 10%, for example 0.5, 1, 2, 3, 5, 6, 7, 8, 9 or 10% usnic acid, said percentages being by weight with respect to the weight of the non-volatile components of the composition namely, in the case of liquid compositions, with respect to the weight of the composition in the solid state (therefore excluding the solvent).

Representative liquid compositions comprise 5 to 30% polymer by weight (with respect to the total weight of the liquid composition) and 0.5 to 10% usnic acid by weight (with respect to the weight of the composition without solvent).

Representative extruded-solid compositions comprise 90 to 99% polymer by weight (with respect to the total weight of the composition) and 0.5 to 10% usnic acid by weight (with respect to the weight of the composition without solvent). The remainder of the composition is essentially constituted by the solvent, advantageously, but not only, THF.

To the compositions of the invention, being in either a extruded-solid or liquid form, one or more additives giving particular performances to the composition are possibly, and in some cases usefully or necessarily, added to the composition and to devices made starting from the composition.

By way of example, it is possible to include a plasticizer, such as for example white pharmaceutical oil, the polymer currently on the market with the trade name K-resin-SBC® and/or other additives, such as adhesive, insulating, blowing, lubricant, antioxidant, process stabilizing, radio-matting, electrically conductive, thermally conductive, antistatic, coloring, releasing, emulsifying, stabilizing, hydrophilic and hydrophobic agents.

Other antimicrobial agents can also be added, for example silver ions or chlorhexidine.

It is also possible to add specific "chemical markers" with the purpose of more easily identifying possible counterfeiting. Such markers are known in the art. Said additives are added to the compositions of the invention, for example at 0.1-30% by weight with respect to the weight of the polymer used in the composition, preferably about 0.1-5%. By way of example antioxidants, preferably at about 0.1%, or multi-walled carbon nanotubes (MWCNT nanotubes), for example at 30%, can be added to the composition.

Still by way of example, it is possible to add a plasticizer to the composition, such as white pharmaceutical oil, at 10÷500% by weight or more, with respect to the weight of the polymer.

According to a preferred embodiment, the compositions in a extruded-solid form essentially comprise the polymer, advantageously the copolymer as defined above and usnic acid, optionally with the addition of small amounts of additives, such as for example antioxidants. In said solid extruded compositions, the amounts of usnic acid are preferably 0.5 to 3%, preferably 1 to 2.5%, whereas the amounts of polymer, advantageously of copolymer as defined above, are preferably 90 to 99% polymer, for example 90-98.5% polymer. Optional additives, for example antioxidants, are present in small amounts, for example 0.05-0.5%, advantageously about 0.2-0.3%.

Other additives are preferably added in the liquid compositions, depending on the use according to the invention.

According to a particularly preferred embodiment, the subject-matter of the invention is a composition comprising the styrene copolymer, advantageously SEEPS, usnic acid and THF (tetrahydrofuran), optionally with the addition of small amounts of additives as reported above, preferably one or more additives selected from antioxidants, releasing agents and pharmaceutical oil. According to a particularly advantageous embodiment, a subject-matter of the invention is a liquid composition comprising:

- a styrene copolymer formed by 0% to 20% low molecular weight SEEPS and 0% to 20% medium molecular weight

SEEPS (provided that at least one of said percentages is different from 0%; said percentages being by weight with respect to the total weight of the composition);

- 40% to 90% solvent (percentage by weight with respect to the total weight of the composition);
- 0.5% to 10% usnic acid (percentages by weight with respect to the total weight of the non-volatile components of the composition);
- 0% to 500% pharmaceutical oil (% by weight with respect to the weight of the copolymer);
- 0.1% to 30% additional additives (% by weight with respect to the weight of the copolymer)

The percentage amounts reported above will be selected depending on desired characteristics for the final device, being one skilled in the art perfectly able to evaluate how to select the right percentages.

By way of example, the higher the amount of solvent the lower the viscosity of the composition will be, the higher the amount of usnic acid, the higher the biocidal capacity will be.

Representative examples are provided in the examples of the experimental section of the present description.

The compositions of the invention are particularly useful for making medicated and medical devices.

According to the present invention, by "medicated and medical devices" instruments are meant which are suitable for the protection of the person, such as for example patches, gloves, condoms etc., but also medical and/or surgical instruments such as inflatable balloons, catheters, infusion tubes, etc. prepared with the compositions of the invention and therefore comprising usnic acid as biocidal agent.

The composition of the invention is useful for preparing said medicated and medical devices.

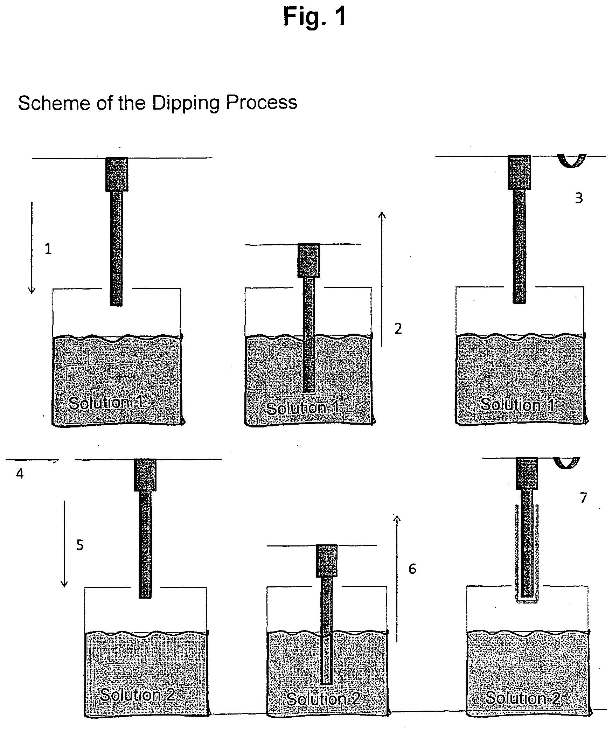

For the implementation of said devices the "dipping" technique can be used. Such a technique is implemented by dipping a punch having the shape of the device to be prepared, thereby obtaining a coating (film) on the punch itself. More in detail and with reference to Figure 1, the process provides for making one first "dipping" in a composition in the liquid form according to the invention, wherein:

1) the punch is lowered with uniform rectilinear motion, preferably at a speed between 500 mm/min and 2000 mm/min; the punch stops in the lowest point and stands still for a set time, for example 2-60 seconds;
2) the punch returns upwards with variable speed depending on the profile of the punch; one skilled in the art is perfectly able to evaluate which is the most suitable speed depending on the shape of the punch;
3) advantageously, the punch must rotate to turn around by 180°. It is important assuring the absolute verticality in order not to create asymmetry to the final semi-finished product. The rotation must be characterized by the rotation speed (rpm) and acceleration leading the punch from standing still to the set speed (rpm).

The punches are generally made of materials inert to the solvent, such as for example Teflon, brass, steel, nylon, porcelain, glass, aluminum, etc., and can be coated by agents easing the release of the device, such as for example calcium carbonate, calcium stearate or silica.

The composition is advantageously thermostated between 2 and 15°C so to maintain the viscosity of the solution constant and limit the solvent evaporation. The repetition of steps 1 to 3 (interspersed by a time period necessary to evaporate the solvent, for example of at least 20 minutes) leads to a punch coating of higher thickness. With the above described technique, it is possible to obtain layers from few hundredths of millimeters up to some millimeters.

The above steps 1 to 3 can also be repeated to add other layers, with compositions equal or different from each other.

In this case the punches, after being dipped in a composition "A", can be for example dipped in a composition "B", thereby obtaining devices with outer, intermediate and inner layers equal or different from each other.

In this way it is possible, for example, to obtain devices with particular characteristics and different from each other, such as:

- devices with hydrophilic inner layer and antibacterial outer layer
- devices with insulating inner layer and electrically conductive outer layer
- devices with insulating inner layer and EMI shielding outer layer
- devices with neutral inner layer and sticky outer layer
- devices with hydrophilic inner layer and outer layer with low permeability to gasses
- devices with adhesive inner layer and neutral outer layer
- devices with inner layer of one color and outer layer of a different color
- devices with neutral inner layer and slippery outer layer when in contact with water
- devices with soft inner layer and rigid outer layer (or vice versa)
- devices with neutral inner layer and blowing outer layer (or vice versa).

Naturally other combinations are possible. The different layers proved to be highly cohesive one to another

and not separable.

If desired or necessary other drugs, in addition to the usnic acid, can be included into the compositions of the invention.

Finally the device is unrolled from the punch to start the step of complete evaporation of the solvent, that can occur slowly at room temperature or forcedly in an oven, for example at about 60°C.

Advantageously, the working environment wherein the process is accomplished, is characterized by a laminar or turbulent isotropic flow of the air surrounding the punch.

The above described process further allows to coat already formed objects with the composition of the invention, for example already extruded medical tubes, gloves, condoms, patches, etc.

Therefore, a further subject-matter of the invention are devices coated with the composition of the invention.

It is understood that the composition must have a viscosity high enough to implement a homogeneous coating that does not drip during the dipping execution and sufficiently low to deposit, at each dipping, a significant layer from the dimensional point of view.

Alternatively, the compositions of the invention can be used to form films by a "deposition" process.

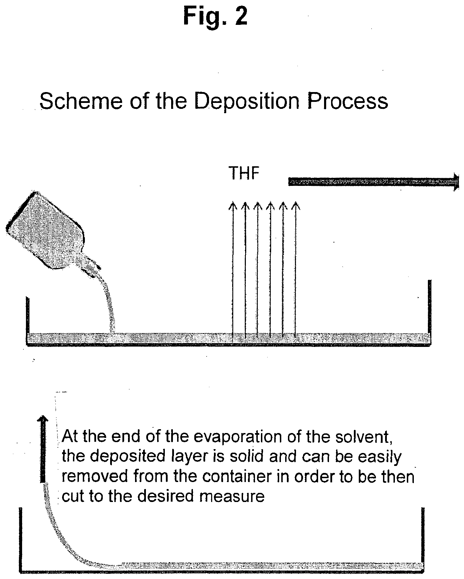

In particular, with reference to Figure 2, the composition of the invention is poured in a suitable container, such as for example a tray and the solvent is let evaporate. Once the evaporation of the solvent is finished, the deposited layer is solid or semi-solid and can easily be removed from the container in order then to be cut at the desired size.

In this way, it is possible to make 0 ShA (very elastic) up to 70 ShD (rigid) films generally usable as patches or 2D coatings. Also in this case multilayer devices can be obtained by overlapping compositions of different solutions. In order to do this, it is sufficient to wait for the evaporation of the solvent and pour another composition over the previous layer, where said new composition could be equal to or different from the previous one.

The container must be made of materials inert to the solvent, such as for example Teflon, brass, steel or nylon.

The evaporated solvent, advantageously THF, can be condensed and re-used for other productions. Such a step allows having less environmental impact in addition to decreasing disposal costs and, obviously, industrial costs of the devices.

Alternative processes wherein the composition of the invention can be used also comprise the nebulization and the layered deposition.

For the nebulization, the container containing the liquid composition can be pressurized (3 to 8 bars) and the nebulized material (through convenient spray nozzle) is directed so that to cover the surface to be treated. A post pre-treatment at 60°C for 4 hours (or more hours) allows totally eliminating the residual ppm of solvent.

For the layered deposition a containing system of the fluid solution can be designed, that can be brought to pressure (1.1 to 8 bars). The container is normally equipped with a fluid outlet nozzle and can move along two orthogonal axes thanks to an electric, electronic or mechanical management. The movements of the container can be set by dedicated software for reiterating the cycle of deposition. The plane on which the fluid deposit occurs can have one or more additional degrees of freedom (rotation and/or translation). The rate of deposition is a function of the evaporation time of the solvent constituting the fluid solution and can vary from 1 second to several minutes.

Depending on the used composition of the invention, make devices having very different hardness can be made, from Sh A 000 up to 75 Sh D, depending on the type, quantity and molecular weight of the copolymer and the optional addition of additives, for example K-resin.

In the same way, the aspect of the devices can space out from the maximum transparency up to an opaque, non-transparent appearance.

The obtained devices can also be extremely elastic (up to 1200% elongation) up to extremely rigid (less than 5% maximum elongation); the elongation is a function of the molecular weight of the copolymer and the amount of pharmaceutical oil in the composition.

A person skilled in the art is certainly able to select the copolymer and additives with the purpose of obtaining devices having the required characteristics.

The so-made devices are a further subject-matter of the invention. Preferred devices are gloves, condoms, patches, dressing films, catheters in general, medical balloons, infusion tubes, urology tubes, gauzes, containing belts, thimbles, menstrual cups, devices for oral hygiene, garments, accomplishment of surfaces for operating rooms, shoe insoles, coatings for subcutaneous electronic or electric devices, generic dentistry equipment, coatings for toothbrushes or equivalent devices for oral hygiene.

The devices of the invention showed to be hypoallergenic and therefore can remain longer in contact with the skin and with mucosae. Said devices also showed to have an optimal shelf-life and therefore can be stored longer. Studies carried out in vitro on different microorganisms demonstrated that the devices of the invention show, thanks to the presence of usnic acid, a very interesting disinfectant capacity and therefore constitute an important progress to the known art. Details of such in vitro assays are provided in the Experimental Section of the present description.

The following examples depict the invention but are not limiting in any way.

The examples demonstrate that the usnic acid can be employed within herein described formulations and

processes in order to obtain semi-finished products, which are obtained, for example but not exclusively, through the above provided processes or mixtures of polymer and usnic acid with bactericidal capacity. In fact, it has been demonstrated that semi-finished products and extruded mixtures of the invention meet the norm ASTM F 2180 ( > 99% killed bacteria).

**Experimental Section**

Example 1

Preparation of medicated gloves according to the invention by the dipping technique

In a glass container (or other material inert to the solvent) 66.7 g granules (or powder or fluffy or flakes, any size, geometry and grain size) of low molecular weight styrene copolymer, are added. 56.8 grams white pharmaceutical oil are then poured within the container and are mixed until total absorption of the oil by the styrene copolymer. Subsequently, 345 grams THF (tetrahydrofuran) solvent are poured and the solvation of the styrene copolymer containing oil is performed by mechanical mixing action (also obtainable with ultrasound). Thus a homogeneous, viscous solution is obtained without any stratification and/or deposition. At the end of the mixing step (time consuming, for example at least 30 minutes) 6.17 grams usnic acid are added. At this point a further mixing for at least 30 minutes is useful so that to disperse usnic acid, no gravimetric deposition or suspension was observed. Thus a homogeneous, stable solution is obtained.

To aid the insertion of the glove on the hand, the inner layer (first dipping) might be made with the same solution but with the final addition of 3-20% PEO (polyethylene oxides) or low molecular weight PEG (polyethylene glycol) of medical grade (50000-500000).

The so-obtained composition is used to prepare medicated gloves by the dipping technique, by using convenient punches made of material inert to the solvent and with low roughness surface, of suitable shape. The so-made gloves are put into an oven at 60°C in order to then be unrolled from the punches. Depending on the thickness of the semi-finished product, the solvent evaporation step can take from few hours to 24 hours in a convection oven at 60°C.

Example 2

Preparation of medicated condoms according to the invention by the dipping technique

In a glass container (or other material inert to the solvent) 66.7 g granules (or powder or fluffy or flakes, any size, geometry and grain size) of low molecular weight styrene copolymer, are added. 56.8 grams white pharmaceutical oil are then poured within the container and are mixed until total absorption of the oil by the copolymer. Subsequently, 370 grams THF (tetrahydrofuran) solvent are poured and the solvation of the copolymer containing oil is performed by mechanical mixing action (also obtainable with ultrasound). Thus a homogeneous, viscous solution is obtained without any stratification and/or deposition. At the end of the mixing step (for example, for at least 30 minutes) 6.17 grams usnic acid are added. At this point a further mixing for at least 30 minutes is necessary so that to disperse usnic acid, no gravimetric deposition or suspension was observed. Thus a homogeneous, stable solution is obtained. The so-obtained composition is used to prepare medicated condoms by the dipping technique, by using convenient punches made of material inert to the solvent and with low roughness surface, of suitable shape. The so-made gloves are put into an oven at 60°C in order to then be unrolled from the punches.

Thus condoms of 0.025 mm thickness are obtained and have optimal mechanical properties and no traces of residual solvent with 2 hours evaporation in an oven at 60°C. To aid the unrolling from the punch, it is possible to cover the condom with a thin layer of $CaCO_3$ (calcium carbonate) or other powdered releasing agents.

Example 3

Preparation of patches according to the invention by the deposition technique

In a glass container (or other material inert to the solvent) 15 grams granules (or powder or fluffy or flakes, any size, geometry and grain size) of medium molecular weight styrene copolymer and 10 grams low molecular weight styrene copolymer, are added. 100 grams white pharmaceutical oil are then poured within the container and are mixed until total absorption of the oil by the copolymer. Subsequently, 335 grams THF (tetrahydrofuran) solvent are poured and the solvation of the copolymer containing oil is performed by mechanical mixing action (also obtainable with ultrasound). Thus a homogeneous, viscous solution is obtained without any stratification and/or deposition. At the end of the mixing step (about at least 30 minutes) 6.25 grams usnic acid are added. At this point a further mixing for at least 30

minutes is useful so that to disperse usnic acid, no gravimetric deposition or suspension was observed. Thus a homogeneous, stable solution is obtained. The so-obtained composition is used to produce medicated patches (film) by the dipping technique, by using a baking tin made of AISI 316 steel. By pouring the obtained solution in the baking tin, the content will homogeneously distribute on the whole surface of the baking tin that must be previously levelled in order to obtain a distribution with constant thickness. The resulting thickness (mm) is obtained by the formula: [(weight of the poured solution) X (100-%THF)/100)]/(0.9xaxb) where a and b are the measures (mm) of the sides of the baking tin. Depending on the thickness of the film, the solvent evaporation step could require up to 24 hours in a convection oven at 60°C.

The obtained film is cut in regular shapes according to market demand and the so-obtained medicated patches are individually packaged.

After usnic acid has been dosed, high molecular weight PEO (900000-7000000) can be added, for example at about 5%-25% (% varies depending on the desired result), to give to the obtained film a distinctive feature of adhesion to the skin. This percentage is referred to the non-volatile component of the solution, therefore namely excluding the amount of THF from the calculation. This further addition will allow the patch to adhere to the skin after being wet with water (or with a physiological solution). In order to detach the patch from the skin, it will be enough to apply a slight tensile stress to the patch, such an operation occurs in a totally atraumatic manner, differently from the classical patches on the market that sometimes have an excessive adhesion thereby making painful the releasing from the skin.

The mixing of copolymer(s) and pharmaceutical oil can also be carried out by a "compounding" process with twin screw extruders, therefore it must not necessarily occur concurrently with the dispersion of the usnic acid and solvent. Substantial differences have not been observed in the devices obtained with the two methods.

Evaluation of the biocidal activity of a device of the invention

To verify the antimicrobial activity of a device of the invention in the form of a patch, through a method that quantitatively evaluated the antimicrobial efficacy of the agents incorporated on polymeric surfaces, a study has been carried out. As a reference, devices of the same material free of antimicrobial agent have been used.

This method entails the inoculation of a melted semi-solid agar (agar slurry) with a standardized culture of microbial cells. A thin layer of inoculated agar slurry is transferred, in triple, over the surfaces to be tested and others used as control. After one or more specified contact times, survived microorganisms are collected from the test substrate upon elution of the inoculum of agar slurry in a neutralizing agent and extracted by a method assuring the complete removal of the inoculum itself from the test surface. Therefore serial dilutions are set, each one seeded upon inclusion in a suitable culture medium. After incubation of the plates in the specified conditions for the used test microorganisms, the number of survived microbial colonies per each dilution is counted and recorded. Therefore, the calculation of the reduction percentage of microorganisms is made by comparing the microorganisms survived on samples of surfaces treated with the antimicrobial agents to those collected on not-treated reference surfaces. Performed tests make reference to the following procedures and operating instructions, subjected to the Sistema di Gestione Qualità certified by ISO 9001 and ISO 13485.

The samples to be tested were squares of 5 x 5 cm size.

The following reagents, materials and laboratory equipment have been used for the test:

- diluent for the preparation of the microbial suspensions: physiological solution with 9 g/l NaCl;
- culture medium for bacteria: Tryptone Soya Agar (TSA);
- culture medium for yeasts: Sabouraud Agar (SAB);
- agar slurry: semi-gel preparation containing 3 g/l agar-agar and 8.5 g/l NaCl;
- recovery / neutralizing broth: solution of Tryptone Soya Broth containing 30 ml/l tween 80, 30 g/l saponin, 1 g/l L-histidine, 3 g/l egg lecithin, 5 g/l sodium thiosulfate;
- ISCO PBI thermostats Cod. SA 05 and SA 06 checked at (31 ± 1)°C;
- MPM INSTRUMENTS thermostated bath Cod. SA 65 checked at (45 ± 1)°C;
- CHIMICA OMNIA thermostated bath Cod. SA 15 checked at (45 ± 1)°C;
- VELP SCIENTIFICA vortex mixer Cod. SA 52;
- PID SYSTEM thermostat Cod. SA 66 checked at (36 ± 1)°C;
- GENESYS 10 spectrophotometer Cod. SA 26;
- VWR ultrasonic tank Cod. SA37;
- various sterile materials (e.g. scissors, pliers etc.).

The preparation of employed media and reagent is carried out according to manufacturer instructions and/or reference method, in accordance with what set forth in the inner operating instructions of Studio Ambiente. Media used in the tests have been checked for fertility and sterility.

Experimental Conditions

The antimicrobial efficacy test has been carried out at the following experimental conditions:
Microbial strains: Pseudomonas aeruginosa ATCC 15442 (gram negative bacteria - indicated as representative in the reference standard), Staphylococcus aureus ATCC6538 (gram positive bacteria - indicated as representative in the reference standard), Staphylococcus aureus MRSA ATCC 43300 (gram positive bacteria - required by the client), Escherichia coli ATCC 10536 (gram negative bacteria - required by the client, representative of strains of enterobacteria),
**[0098]** Candida albicans ATCC 10231 (yeasts - required by the client).
The strains are managed according to what specified in the reference, internal, operating instruction. The incubation conditions used for the test strains are detailed in the table below.

Tab. 1 Test strain Temperature (°C)

| Time (h) | | |
|---|---|---|
| Test Strain | Temperature (°C) | Time (h) |
| Candida albicans | (30 ± 1)°C | 48 h |
| Escherichia coli | (36 ± 1)°C | 48 h |
| Pseudomonas aeruginosa | (36 ± 1)°C | 48 h |
| Staphylococcus aureus | (36 ± 1)°C | 48 h |
| Staphylococcus aureus MRSA | (36 ± 1)°C | 48 h |
| The contact times have been the following: 24 h (1 day), 48 h (2 days), 72 h (3 days). | | |

Test description

Each microbial strain has been transplanted on slant of suitable medium for 24 hours and then diluted in physiological solution until reaching a concentration, assessed through spectrophotometric reading, between 1-5 x 108 CFU/ml. The number of microbial cells in each suspension has been determined through dilutions scaled by 10 in physiological solution, up to 10-6. From this dilution two aliquots of 1 ml have been withdrawn and seeded upon inclusion in a suitable medium (TSA for bacteria and SAB for yeasts). After incubation and counting of colonies developed on plates, the number of colony forming units per ml (CFU/ml) in each suspension has been determined.
1.0 ml of each microbial suspension has been seeded in 100 ml of agar slurry, kept melted at the temperature of 45°C in order to obtain a final concentration of microbial cells in each agar slurry between 1-5 x 106 CFU/ml.
Test and reference devices have been prepared by inserting, in a conveniently identified plate, 3 pieces for a contact time defined above. They have been previously moistened with physiological solution to avoid the slurry to penetrate and aid the uniform dispersion thereof on the sample.
1.0 ml of inoculated agar slurry has been transferred on each test and control sample prepared for each test suspensions. The inoculation has been carried out with such angle and rate to assure the slow imbibition of the sample itself, thereby avoiding the loss of solution. After allowing the inoculum of agar slurry to jellify, the samples have been placed in the incubators at a temperature suitable for the development of microbial strains for the defined contact times. In order to avoid the drying of the inoculum of agar slurry within the thermostats, maintaining the humidity at a level higher than 75% has been assured by using a bowl containing water.
At each defined contact time the samples of reference devices, treated or not treated, have been removed from the Petri plates and transferred in a flask containing neutralizing broth in such a volume to give a 1:10 or 1: 100 dilution of the initial inoculum.
The flasks underwent sonication for 1 minute, followed by a subsequent mechanical mixing by using the vortex so that to assure the complete release of the agar slurry from the sample.
Therefore the neutralizing broth has been subjected to serial 1: 10 dilutions, each one seeded upon inclusion in a medium suitable for the development of the specific microbial strain.
The sample has been seeded upon inclusion in melted medium, in order to determine the efficacy of releasing from the treated surface.
After incubation, the number of colonies developed per each prepared dilution has been counted and recorded by calculating the number of microorganisms (CFU/ml) survived for each suspension and contact time.

Calculation and expression of the results

The results have been expressed in the form of percentage reduction of the microbial contamination of the treated device sample compared to the not treated one, according to what set forth in the reference standard. The geometric mean of the number of microorganisms collected in the three repetitions made for the devices treated and not treated with antimicrobial agent has been calculated; therefore, the percentage difference between the anti-logarithm of the geometric mean of the control sample and the anti-logarithm of the geometric mean of the treated sample, has been calculated.

$$\text{Geometric mean} = (\text{LogR1} + \text{LogR2} + \text{LogR3})/3$$

Where R1/2/3 = total number of collected microorganisms after exposure to the test substance or to control and incubation (repetition 1/2/3).

$$\text{Percentage reduction} = (a-b)x100/a$$

Where:

a = anti-logarithm of the geometric mean of the not treated, reference device
b = anti-logarithm of the geometric mean of the treated device

Criteria of validity of the assay

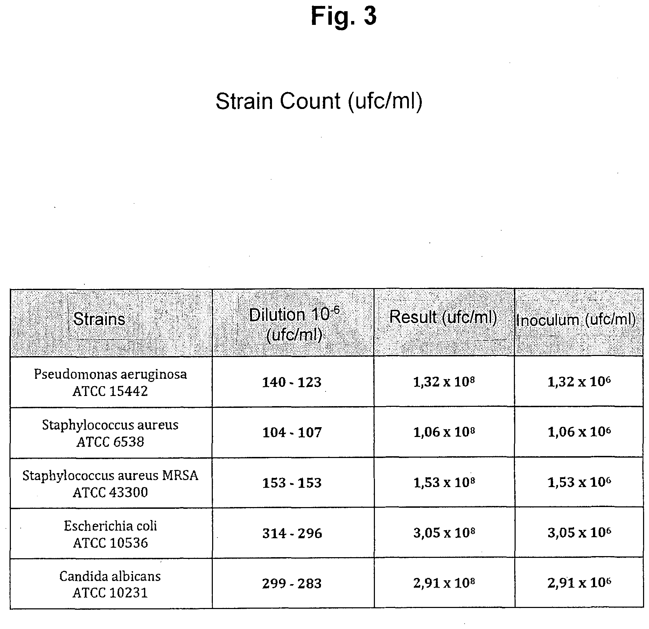

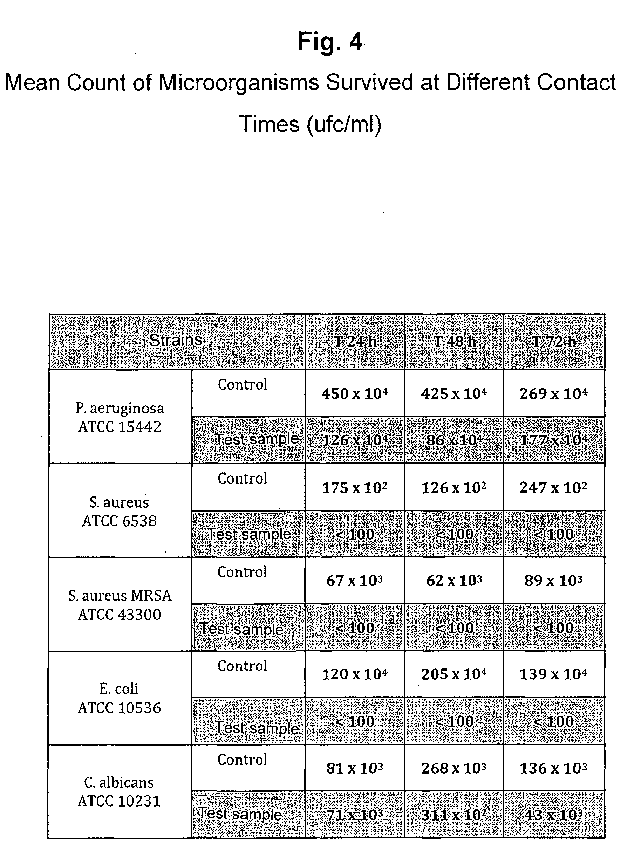

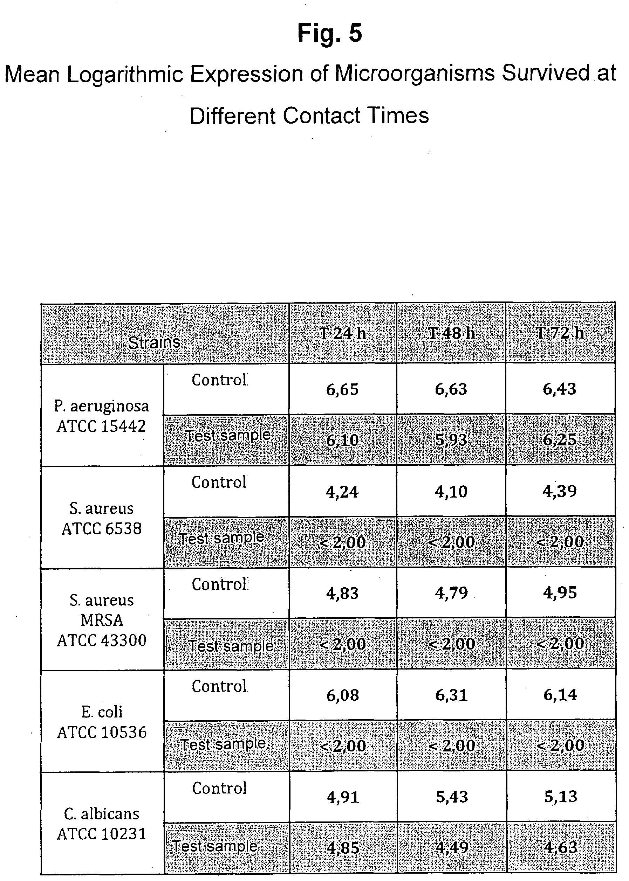

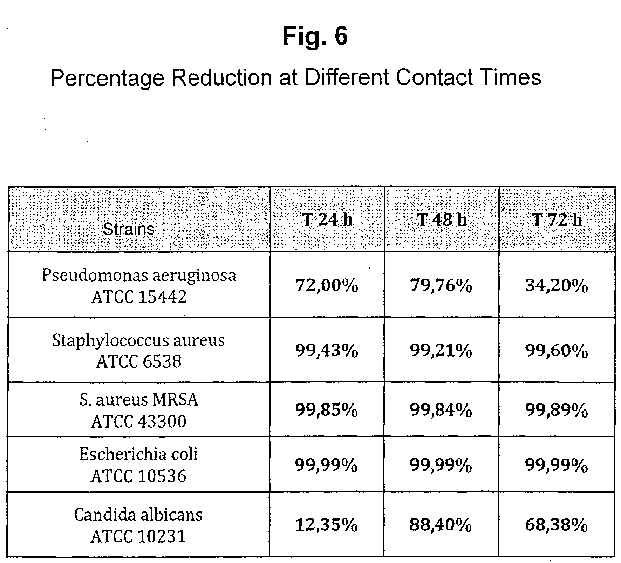

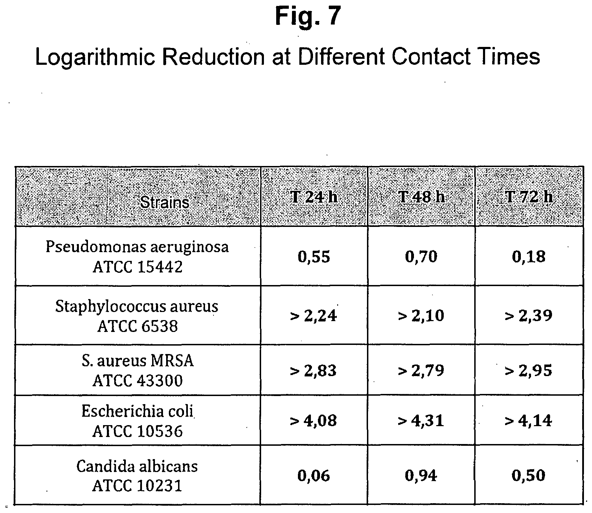

The assay is considered valid when the initial collection of microorganisms is equal to or higher than 104 CFU/ml. The device sample treated with the tested usnic acid is considered effective when the percentage reduction of the microbial contamination obtained therewith, compared to the not treated device, is equal to or higher than 99%.
The results are set forth in Figures 3 to 7 attached to the present description.

Conclusions

Based on the obtained results, once the test validity criteria have been respected for the test samples in the adopted experimental conditions, the following conclusions can be drawn:

- strains of gram positive bacteria (Staphylococcus aureus and Staphylococcus aureus MRSA and Staphylococcus epidermidis): the samples proved to be very effective with a reduction higher than 99% with contact time from 24 hours up to 72 hours;
- strain of gram negative bacteria representative of enterobacteria (Escherichia coli): the samples proved to be very effective with a reduction higher than 99% with contact time from 24 hours up to 72 hours;
- strain of gram negative bacteria (Pseudomonas aeruginosa): the samples proved to be effective enough showing an abatement approximately equal to 70% at 24 and 48 hours;
- strain of yeasts (Candida albicans): the samples proved to be effective enough showing an abatement higher than 60% at 48 and 72 hours.

Example 4

Preparation of solid composition for extrusion

For this study a "compound" of hardness 32 ShD formulated for extruding tubes presenting a good resistance against "kinking", which maintain a good transparency also with the addition of usnic acid and are steam sterilizable (121°C and 134°C), has been made.
The two tested formulations were so composed:

| Description | | Test A | Test B |
|---|---|---|---|
| USNIC ACID NLT 98% | % | 1.2 | 2.4 |

(continued)

| Description | | Test A | Test B |
|---|---|---|---|
| KRATON G 1645 MO | % | 54.8 | 54.2 |
| RC 737 MO | % | 43.8 | 43.2 |
| IRGAFOS 168 FF | % | 0.1 | 0.1 |
| IRGANOX 1010 FF | % | 0.1 | 0.1 |

The compounding process has been made through a twin screw extruder of 30 mm diameter, 36 diameters with the following set up:

| Screw speed (rpm) | T1 (°C) | T2 (°C) | T3 (°C) | T4 (°C) | T5 (°C) | T6 (°C) | T7 (°C) |
|---|---|---|---|---|---|---|---|
| 650 | 80 | 131 | 170 | 170 | 170 | 110 | 195 |

The use of an infrared reader for reading the temperature of the melted sample is preferred as a wrong positioning of the thermocouple, for reading the melted sample, could return temperatures of the melted sample different from the actual one. In such a study we assured that the maximum temperature of the melted sample was less than the melting temperature of the usnic acid.

In order to understand and certify how good the formulation and compounding process were, flat surfaces have been extruded with a 19 mm extruder, 25 diameters.

| Screw speed (rpm) | T1 (°C) | T2 (°C) | T3 (°C) | T4 (°C) |
|---|---|---|---|---|
| 70 | 90 | 140 | 160 | 180 |

It is essential, also for this process, to continuously monitor the temperature of the melted sample by imposing the actual temperature of the melted sample being less than 200°C.

Characterization of Test Compound A:

| | | |
|---|---|---|
| Shore Hardness A - after 3 sec. | 84 | ShA |
| Melt Flow Index | 10 | gr/ 10’ |
| Specific Gravity | 0,886 | gr/cc |
| Elongation at Break | 750 | % |
| Breaking load | 10.7 | MPa |
| Tear Resistance Without Notching | 55.5 | MPa |
| 100% Modulus | 3.5 | MPa |
| 300% Modulus | 4.9 | MPa |

Characterization of Test Compound B:

| | | |
|---|---|---|
| Shore Hardness A - after 3 sec. | 88 | ShA |
| Melt Flow Index | 5.5 | gr/ 10’ |
| Specific Gravity | 0,885 | gr/cc |

(continued)

| | | |
|---|---|---|
| Elongation at Break | 855 | % |
| Breaking load | 15.8 | MPa |
| Tear Resistance Without Notching | 62.2 | MPa |
| 100% Modulus | 4.3 | MPa |
| 300% Modulus | 5.5 | MPa |

On the strips extruded with such a compound, the norm ASTM F 2180 has been performed on the following bacterial cultures after a contact time of 24 and 72 hours, with the following results:

| Strains | Sample | T 24 h | T 72 h |
|---|---|---|---|
| S. aureus MRSA ATCC 43300 | Devices 1.2% usnic acid | 98.48% | 99.99% |
| | Devices 2.4% usnic acid | 99.05% | 99.99% |
| Escherichia coli ATCC 10536 | Devices 1.2% usnic acid | 18.72% | 99.96% |
| | Devices 2.4% usnic acid | 16.82% | 99.97% |

Thus it is demonstrated that the usnic acid can be employed within formulations and processes as herein described, in order to obtain extruded semi-finished products with bactericidal capability. The semi-finished products obtained by extrusion, depending on the contact time, pass the norm ASTM F 2180 (>99% killed bacteria).

The IR spectra performed on blanks extruded with compound with usnic acid and pure usnic acid, confirmed the presence of usnic acid on the surface of the extruded ones; in fact, wavelengths in the spectrum of usnic acid only and those of the compound with usnic acid are the same, demonstrating that the usnic acid is present on the surface of the extruded strips without signs of post- extrusion degradation (compounding).

Example 5 (comparative)

Mixtures of polyurethane and usnic acid in solution of one solvent selected from tetrahydrofuran, cyclohexanone and methylethylketon, have been prepared.

Performed tests lead to negative outcomes because the solvation didn't occur homogeneously and completely and, above all, inserting usnic acid in these mixtures didn't lead to a stable and homogeneous dispersion (namely to a concentration of usnic acid equal and constant in each sample of volume of the solution). Instead, the deposition over time of usnic acid on the bottom of the container has been observed. Therefore such mixtures cannot assure a bactericidal performance to the user because a homogeneous presence of usnic acid within the mixture is not assured.

## Claims

1. Liquid or extruded-solid composition comprising usnic acid and a polymer selected from PVC, a copolymer selected from SBS, SEBS, SEP, SEPS, SEBS, SEEPS, SBC and mixtures of said copolymers.

2. Composition according to claim 1, **characterized in that** said copolymer is selected from SBS, SEBS, SEP, SEPS, SEEPS and mixtures thereof.

3. Composition according to claim 1 or 2, **characterized in that** said copolymer is SEEPS.

4. Composition according to any one of claims 1 to 3, **characterized in that** it is in a solid form.

5. Composition according to claim 4, **characterized by** comprising 0.5 to 10% usnic acid and 90 to 99% polymer.

6. Composition according to any one of claims 1 to 4, **characterized by** being in a liquid form dispersed in an organic solvent.

7. Composition according to claim 6, **characterized by** comprising 1 to 40% by weight of polymer with respect to the total weight of the composition.

8. Composition according to any one of claims 6 or 7, **characterized by** comprising 5 to 30% polymer (with respect to the total weight of the liquid composition) and 0.5 to 10% usnic acid (with respect to the weight of the composition without solvent).

9. Process for preparing a medicated device, comprising dipping a punch having the shape of said device in a composition according to one of claims 6 to 8, extracting said punch, isolating said so-formed device, and evaporating the solvent.

10. Process according to claim 9, comprising letting the solvent evaporate and dipping again the punch in a composition according to any one of claims 6 to 8, said composition being equal to or different from the previous one.

11. Process for preparing a medicated device, comprising pouring a composition according to any one of claims 6 to 8 on a horizontal support, letting the solvent evaporate thereby obtaining a film and, in case, portioning said film.

12. Process according to claim 11, comprising letting the solvent evaporate and then pouring a composition according to any one of claims 6 to 8 on the previously obtained film, said composition being equal to or different from the previous one.

13. Process for preparing the composition according to claims 4 or 5, **characterized by** comprising extruding a mixture of usnic acid and the polymer.

14. Medicated or medical device obtained by the composition according to any one of claims 1 to 10 or by the process of any one of claims 1 to 10.

15. Device according to claim 14 selected from gloves, condoms, patches, dressing films, catheters in general, medical balloons, infusion tubes, urology tubes, gauzes, containing belts, thimbles, menstrual cups, devices for oral hygiene, garments, accomplishment of surfaces for operating rooms, shoe insoles, coatings for subcutaneous electronic or electric devices, dentistry equipment, coatings for toothbrushes or equivalent devices for oral hygiene.

**Patentansprüche**

1. Flüssige oder extrudiert-feste Zusammensetzung, umfassend Usninsäure und ein Polymer, ausgewählt aus PVC, einem Copolymer, ausgewählt aus SBS, SEBS, SEP, SEPS, SEBS, SEEPS, SBC und Mischungen dieser Copolymere.

2. Zusammensetzung nach Anspruch 1, **dadurch gekennzeichnet, dass** das Copolymer aus SBS, SEBS, SEP, SEPS, SEEPS und Mischungen davon ausgewählt ist.

3. Zusammensetzung nach Anspruch 1 oder 2, **dadurch gekennzeichnet, dass** das Copolymer SEEPS ist.

4. Zusammensetzung nach einem der Ansprüche 1 bis 3, **dadurch gekennzeichnet, dass** sie in fester Form vorliegt.

5. Zusammensetzung nach Anspruch 4, **dadurch gekennzeichnet, dass** sie 0,5 bis 10 % Usninsäure und 90 bis 99 % Polymer umfasst.

6. Zusammensetzung nach einem der Ansprüche 1 bis 4, **dadurch gekennzeichnet, dass** sie in einer flüssigen Form in einem organischen Lösungsmittel dispergiert vorliegt.

7. Zusammensetzung nach Anspruch 6, **dadurch gekennzeichnet, dass** sie 1 bis 40 Gew.-% Polymer bezogen auf das Gesamtgewicht der Zusammensetzung umfasst.

8. Zusammensetzung nach einem der Ansprüche 6 oder 7, **dadurch gekennzeichnet, dass** sie 5 bis 30 % Polymer (bezogen auf das Gesamtgewicht der flüssigen Zusammensetzung) und 0,5 bis 10 % Usninsäure (bezogen auf das Gewicht der Zusammensetzung ohne Lösungsmittel) umfasst.

**9.** Verfahren zur Herstellung einer medizinischen Vorrichtung, umfassend das Eintauchen eines Stempels mit der Form der Vorrichtung in eine Zusammensetzung nach einem der Ansprüche 6 bis 8, Herausziehen des Stempels, Isolieren der so gebildeten Vorrichtung und Verdampfen des Lösungsmittels.

**10.** Verfahren nach Anspruch 9, umfassend das Verdampfenlassen des Lösungsmittels und erneutes Eintauchen des Stempels in eine Zusammensetzung nach einem der Ansprüche 6 bis 8, wobei die Zusammensetzung gleich oder verschieden von der vorherigen ist.

**11.** Verfahren zur Herstellung einer medizinischen Vorrichtung, umfassend das Gießen einer Zusammensetzung nach einem der Ansprüche 6 bis 8 auf einen horizontalen Träger, Verdampfenlassen des Lösungsmittels, wodurch ein Film erhalten wird, und gegebenenfalls Portionieren des Films.

**12.** Verfahren nach Anspruch 11, umfassend das Verdampfenlassen des Lösungsmittels und anschließendes Gießen einer Zusammensetzung nach einem der Ansprüche 6 bis 8 auf die zuvor erhaltene Folie, wobei die Zusammensetzung gleich oder verschieden von der vorherigen ist.

**13.** Verfahren zur Herstellung der Zusammensetzung nach Anspruch 4 oder 5, **dadurch gekennzeichnet, dass** es das Extrudieren einer Mischung aus Usninsäure und dem Polymer umfasst.

**14.** Medikament oder medizinisches Gerät, erhalten durch die Zusammensetzung nach einem der Ansprüche 1 bis 10 oder das Verfahren nach einem der Ansprüche 1 bis 10.

**15.** Vorrichtung nach Anspruch 14, ausgewählt aus Handschuhen, Kondomen, Pflastern, Verbandsfolien, Kathetern im Allgemeinen, medizinischen Ballons, Infusionsschläuchen, Urologieschläuchen, Mullbinden, Haltegurten, Fingerhüten, Menstruationstassen, Vorrichtungen für die Mundhygiene, Kleidungsstücken, Ausstattung von Oberflächen für Operationssäle, Schuheinlagen, Beschichtungen für subkutane elektronische oder elektrische Geräte, zahnmedizinische Geräte, Beschichtungen für Zahnbürsten oder gleichwertige Geräte für die Mundhygiene.

## Revendications

**1.** Composition liquide ou extrudée-solide comprenant de l'acide usnique et un polymère choisi parmi le PVC, un copolymère choisi parmi le SBS, le SEBS, le SEP, le SEPS, le SEBS, le SEEPS, le SBC et les mélanges desdits copolymères.

**2.** Composition selon la revendication 1, **caractérisée en ce que** ledit copolymère est choisi parmi le SBS, le SEBS, le SEP, le SEPS, le SEEPS et leurs mélanges.

**3.** Composition selon la revendication 1 ou 2, **caractérisée en ce que** ledit copolymère est un SEEPS.

**4.** Composition selon l'une quelconque des revendications 1 à 3, **caractérisée en ce qu'**elle se présente sous forme solide.

**5.** Composition selon la revendication 4, **caractérisée en ce qu'**elle comprend de 0,5 à 10 % d'acide usnique et de 90 à 99 % de polymère.

**6.** Composition selon l'une quelconque des revendications 1 à 4, **caractérisée en ce qu'**elle se présente sous forme liquide dispersée dans un solvant organique.

**7.** Composition selon la revendication 6, **caractérisée en ce qu'**elle comprend de 1 à 40 % en poids de polymère par rapport au poids total de la composition.

**8.** Composition selon l'une quelconque des revendications 6 ou 7, **caractérisée en ce qu'**elle comprend 5 à 30 % de polymère (par rapport au poids total de la composition liquide) et 0,5 à 10 % d'acide usnique (par rapport au poids de la composition sans solvant).

**9.** Procédé de préparation d'un dispositif médicamenteux, comprenant le trempage d'un poinçon ayant la forme dudit dispositif dans une composition selon l'une des revendications 6 à 8, l'extraction dudit poinçon, l'isolement dudit

dispositif ainsi formé et l'évaporation du solvant.

**10.** Procédé selon la revendication 9, comprenant laisser évaporer le solvant et tremper à nouveau le poinçon dans une composition selon l'une quelconque des revendications 6 à 8, ladite composition étant égale ou différente de la précédente.

**11.** Procédé de préparation d'un dispositif médicamenteux, comprenant le versement d'une composition selon l'une quelconque des revendications 6 à 8 sur un support horizontal, laisser évaporer le solvant pour obtenir un film et, le cas échéant, le portionnement dudit film.

**12.** Procédé selon la revendication 11, comprenant laisser évaporer le solvant puis verser une composition selon l'une quelconque des revendications 6 à 8 sur le film précédemment obtenu, ladite composition étant égale ou différente de la précédente.

**13.** Procédé de préparation de la composition selon les revendications 4 ou 5, **caractérisé en ce qu'**il comprend l'extrusion d'un mélange d'acide usnique et de polymère.

**14.** Dispositif médicamenteux ou médical obtenu par la composition selon l'une quelconque des revendications 1 à 10 ou par le procédé de l'une quelconque des revendications 1 à 10.

**15.** Dispositif selon la revendication 14 choisi parmi des gants, des préservatifs, des patchs, des films de pansement, des cathéters en général, des ballons médicaux, des tubes de perfusion, des tubes d'urologie, des gazes, des ceintures de contention, des chapes des coupes menstruelles, des dispositifs pour l'hygiène buccale, des vêtements, une réalisation de surfaces pour salles d'opération, des semelles de chaussures, des revêtements pour dispositifs électroniques ou électriques sous-cutanés, un matériel de dentisterie, des revêtements pour brosses à dents ou des dispositifs équivalents pour l'hygiène buccale.

# Fig. 1

Scheme of the Dipping Process

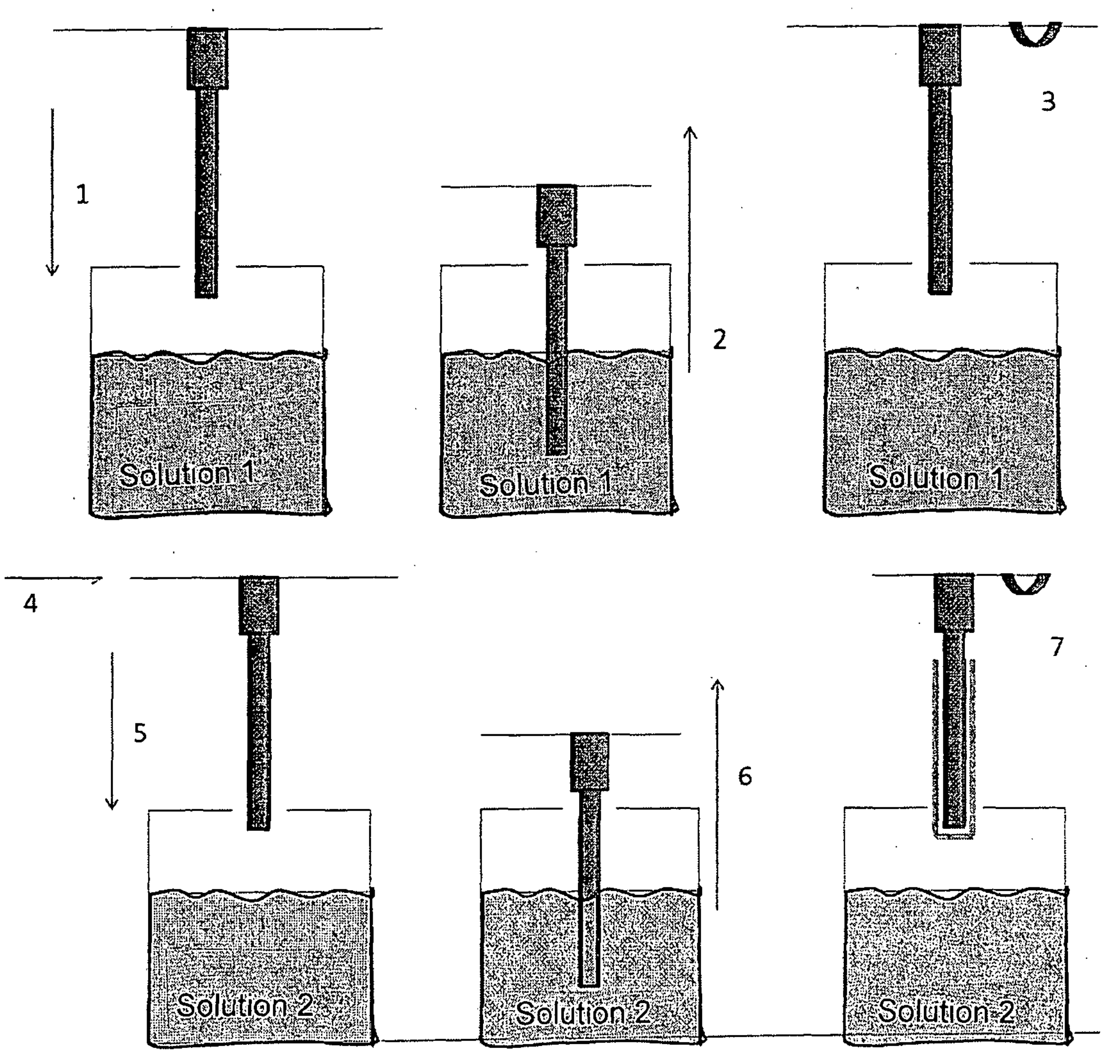

Fig. 2

Scheme of the Deposition Process

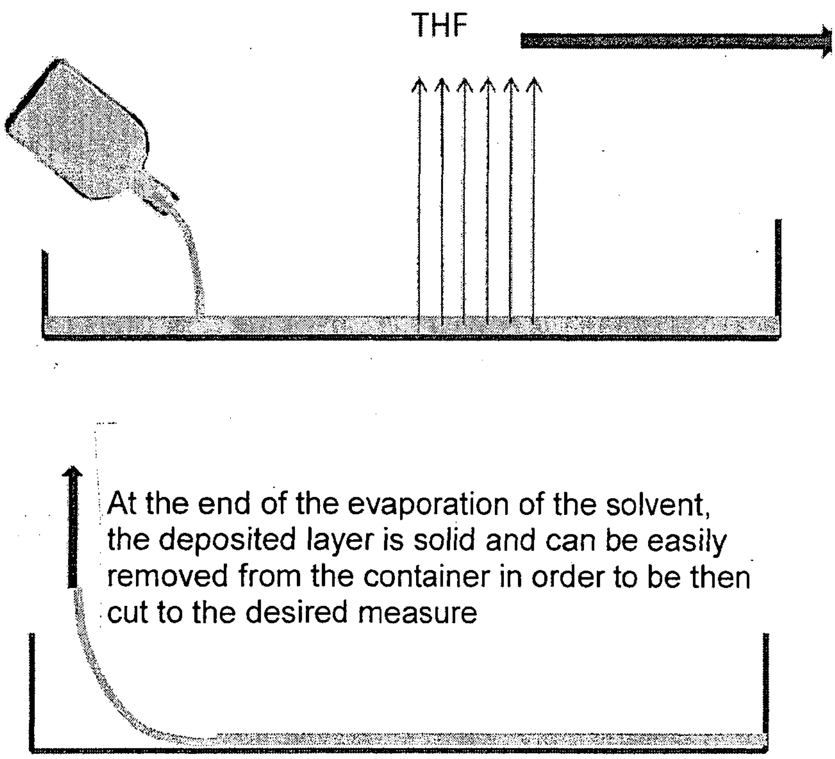

# Fig. 3

## Strain Count (ufc/ml)

| Strains | Dilution $10^{-6}$ (ufc/ml) | Result (ufc/ml) | Inoculum (ufc/ml) |
|---|---|---|---|
| Pseudomonas aeruginosa ATCC 15442 | **140 - 123** | **$1{,}32 \times 10^8$** | **$1{,}32 \times 10^6$** |
| Staphylococcus aureus ATCC 6538 | **104 - 107** | **$1{,}06 \times 10^8$** | **$1{,}06 \times 10^6$** |
| Staphylococcus aureus MRSA ATCC 43300 | **153 - 153** | **$1{,}53 \times 10^8$** | **$1{,}53 \times 10^6$** |
| Escherichia coli ATCC 10536 | **314 - 296** | **$3{,}05 \times 10^8$** | **$3{,}05 \times 10^6$** |
| Candida albicans ATCC 10231 | **299 - 283** | **$2{,}91 \times 10^8$** | **$2{,}91 \times 10^6$** |

# Fig. 4

Mean Count of Microorganisms Survived at Different Contact Times (ufc/ml)

| Strains | | T 24 h | T 48 h | T 72 h |
|---|---|---|---|---|
| P. aeruginosa ATCC 15442 | Control | **450 x $10^4$** | **425 x $10^4$** | **269 x $10^4$** |
| | Test sample | **126 x $10^4$** | **86 x $10^4$** | **177 x $10^4$** |
| S. aureus ATCC 6538 | Control | **175 x $10^2$** | **126 x $10^2$** | **247 x $10^2$** |
| | Test sample | **< 100** | **< 100** | **< 100** |
| S. aureus MRSA ATCC 43300 | Control | **67 x $10^3$** | **62 x $10^3$** | **89 x $10^3$** |
| | Test sample | **< 100** | **< 100** | **< 100** |
| E. coli ATCC 10536 | Control | **120 x $10^4$** | **205 x $10^4$** | **139 x $10^4$** |
| | Test sample | **< 100** | **< 100** | **< 100** |
| C. albicans ATCC 10231 | Control | **81 x $10^3$** | **268 x $10^3$** | **136 x $10^3$** |
| | Test sample | **71 x $10^3$** | **311 x $10^2$** | **43 x $10^3$** |

# Fig. 5

Mean Logarithmic Expression of Microorganisms Survived at Different Contact Times

| Strains | | T 24 h | T 48 h | T 72 h |
|---|---|---|---|---|
| P. aeruginosa ATCC 15442 | Control | 6,65 | 6,63 | 6,43 |
| | Test sample | 6,10 | 5,93 | 6,25 |
| S. aureus ATCC 6538 | Control | 4,24 | 4,10 | 4,39 |
| | Test sample | < 2,00 | < 2,00 | < 2,00 |
| S. aureus MRSA ATCC 43300 | Control | 4,83 | 4,79 | 4,95 |
| | Test sample | < 2,00 | < 2,00 | < 2,00 |
| E. coli ATCC 10536 | Control | 6,08 | 6,31 | 6,14 |
| | Test sample | < 2,00 | < 2,00 | < 2,00 |
| C. albicans ATCC 10231 | Control | 4,91 | 5,43 | 5,13 |
| | Test sample | 4,85 | 4,49 | 4,63 |

# Fig. 6

Percentage Reduction at Different Contact Times

| Strains | T 24 h | T 48 h | T 72 h |
|---|---|---|---|
| Pseudomonas aeruginosa ATCC 15442 | 72,00% | 79,76% | 34,20% |
| Staphylococcus aureus ATCC 6538 | 99,43% | 99,21% | 99,60% |
| S. aureus MRSA ATCC 43300 | 99,85% | 99,84% | 99,89% |
| Escherichia coli ATCC 10536 | 99,99% | 99,99% | 99,99% |
| Candida albicans ATCC 10231 | 12,35% | 88,40% | 68,38% |

# Fig. 7

Logarithmic Reduction at Different Contact Times

| Strains | T 24 h | T 48 h | T 72 h |
|---|---|---|---|
| Pseudomonas aeruginosa ATCC 15442 | 0,55 | 0,70 | 0,18 |
| Staphylococcus aureus ATCC 6538 | > 2,24 | > 2,10 | > 2,39 |
| S. aureus MRSA ATCC 43300 | > 2,83 | > 2,79 | > 2,95 |
| Escherichia coli ATCC 10536 | > 4,08 | > 4,31 | > 4,14 |
| Candida albicans ATCC 10231 | 0,06 | 0,94 | 0,50 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012123803 A

### Non-patent literature cited in the description

- **FRANCOLINI et al.** *Antimicrobial Agents and Chemotherapy,* November 2004, 4360-4365